# EUROPÄISCHE PATENTSCHRIFT

(11) **EP 3 672 142 B1**
(45) Veröffentlichungstag und Bekanntmachung des Hinweises auf die Patenterteilung: **21.04.2021**
(21) Anmeldenummer: 18214594.6
(22) Anmeldetag: 20.12.2018
(51) Int. Cl.: H04L 9/08, H04L 9/14, H04L 9/32

(54) **VERFAHREN UND SYSTEM ZUR SICHEREN ÜBERTRAGUNG EINES DATENSATZES**
METHOD AND SYSTEM FOR SECURELY TRANSFERRING A DATA SET
PROCÉDÉ ET SYSTÈME DE TRANSMISSION SÉCURISÉE D'UN ENSEMBLE DE DONNÉES

(43) Veröffentlichungstag der Anmeldung: 24.06.2020
(73) Patentinhaber: Siemens Healthcare GmbH, 91052 Erlangen (DE)
(72) Erfinder: Fries, Steffen, 85598 Baldham (DE); Rosenbaum, Ute, 87437 Kempten (DE)

(56) Entgegenhaltungen:
- US-A1- 2017 171 178
- DONG ZHEMING ET AL: "Security Enhanced Anonymous Remote User Authentication and Key Agreement for Cloud Computing", 2014 IEEE 17TH INTERNATIONAL CONFERENCE ON COMPUTATIONAL SCIENCE AND ENGINEERING, IEEE, 19. Dezember 2014 (2014-12-19), Seiten 1746-1751, XP032730242, DOI: 10.1109/CSE.2014.320 [gefunden am 2015-01-26]

## Beschreibung

Die Erfindung betrifft ein Verfahren und ein System zur sicheren Übertragung eines Datensatzes, insbesondere eines medizinischen Datensatzes, zwischen einem Server und einem Benutzergerät mittels eines Cloud-Dienstes, sowie eine entsprechende medizintechnische Anlage.

In immer mehr Anwendungsfällen werden Datendienste als Cloud-Anwendungen genutzt, um den Zugriff auf Daten außerhalb eines privaten, geschlossenen Netzwerkes oder auch die Verarbeitung von in der Cloud befindlichen Daten zu realisieren. Beispiele hierfür sind die Verarbeitung von Messdaten durch Smart Meter in der Cloud, z.B. durch einen Messstellenbetreiber. Ein weiteres Beispiel ist die Auswertung von Parametern von bildgebenden Verfahren im Gesundheitswesen. In beiden Fällen ist eine Anonymisierung oder Pseudonymisierung der Daten erforderlich, um den Bezug zu einer bestimmten Person unkenntlich zu machen.

Es sind verschiedenen Verfahren zur Übertragung sensitiver Daten bekannt, von denen einige im Folgenden näher erläutert werden.

Ein unter dem Namen "S/MIME" bekanntes Verfahren ermöglicht den Email-Versand von verschlüsselten Daten. S/MIME setzt hierbei auf eine zweistufige Verschlüsselung. Die eigentliche Nachricht wird zum Schutz der Vertraulichkeit symmetrisch verschlüsselt. Der dazu verwendete Schlüssel wird dann mit dem öffentlichen Schlüssel des Empfängers (asymmetrisch) verschlüsselt. Damit ist sichergestellt, dass nur der Empfänger die Email entschlüsseln kann, der auch im Besitz des korrespondierenden privaten Schlüssels ist.

Die nach den Anfangsbuchstaben der Nachnamen der Erfinder benannte "RSA-Verschlüsselung" ermöglicht die Verschlüsselung von Daten mit dem öffentlichen Schlüssel eines Empfängers.

Die hierbei verwendete asymmetrische Verschlüsselung ist zeitaufwendig, was zur Folge hat, dass in der Regel kombinierte Verfahren (wie zuvor bei S/MIME beschrieben) zum Einsatz kommen. RSA Encryption wird daher insbesondere zum Schlüsselmanagement angewendet. Ein Beispiel ist TLS (IETF RFC 5246), ein Sicherheitsprotokoll, das bei vielfach bei webbasierten Anwendungen genutzt wird.

Die "Diffie-Hellman-Schlüsselaushandlung" ist ein Verfahren zur Etablierung eines symmetrischen Schlüssels, der dann zur Sicherung von Massendaten genutzt werden kann. Hierbei erzeugen Sender und Empfänger jeweils ein Schlüsselpaar, dessen jeweiligen öffentlichen Schlüssel veröffentlicht werden können. Die jeweiligen geheimen Schlüssel verbleiben beim Sender bzw. beim Empfänger. Der Sender kann mit dem öffentlichen Empfängerschlüssel und seinem geheimen Senderschlüssel eine Botschaft für den Empfänger generieren, die der Empfänger unter Nutzung des öffentlichen Senderschlüssels und seines geheimen Empfängerschlüssels wieder extrahieren kann. Es sind weiterhin Zertifikate bekannt (ECDSA Zertifikate), deren öffentlicher ECDSA Schlüssel gleichzeitig als öffentlicher Diffie Hellman Schlüssel genutzt werden kann.

Ein unter der Bezeichnung "ISO/IEC 15118" bekannter Standard beschreibt ein Verfahren für den Kommunikationsaustausch zwischen einer Ladesäule und einem Elektrofahrzeug. Ein Teil der ausgetauschten Informationen umfasst ein im Backend generiertes Schlüsselpaar, das mittels einer Nachricht zum Elektrofahrzeug transportiert werden kann. Das Auto besitzt ein ECDSA basiertes Schlüsselpaar, das im Backend bekannt ist. Im Backend wird der öffentliche Schlüssel im ECDSA Zertifikat als statischer Diffie Hellman Schlüssel behandelt. Will das Backend das zentral erzeugte Schlüsselpaar gesichert zum Elektrofahrzeug übertragen, erzeugt es ein eigenes Diffie Hellman Schlüsselpaar und generiert aus dem öffentlichen Schlüssel des Elektrofahrzeugs und dem eigenen geheimen Schlüssel das Diffie Hellman Geheimnis und verschlüsselt mit diesem das Schlüsselpaar, das geschützt zum Elektrofahrzeug transportiert werden soll. Im Anschluss wird das verschlüsselte Schlüsselpaar und der öffentliche Diffie Hellmann Schlüssel des Backends signiert an das Elektrofahrzeug geschickt. Dies kann nun mittels seines privaten Diffie Hellman Schlüssels und dem öffentlichen Schlüssel des Backends ebenfalls das Diffie Hellman Geheimnis erzeugen und das Schlüsselpaar entschlüsseln und installieren. Diese Lösung funktioniert aber nur, wenn beide Parteien den zugrundeliegenden Zertifikaten, die zur Signatur verwendet wurden, vertrauen. Dieses Vertrauen wird im Allgemeinen durch eine gemeinsame oder für beide Partner vertrauenswürdige Zertifizierungsstelle erreicht Weitere Ansätze aus dem Stand der Technik sind im Dokument "Security Enhanced Anonymous Remote User Authentication and Key Agreement for Cloud Computing", Autoren DONG ZHEMING ET AL, 2014 IEEE 17TH INTERNATIONAL CONFERENCE ON COMPUTATIONAL SCIENCE AND ENGINEERING, IEEE, 19. Dezember 2014 (2014-12-19), Seiten 1746-1751, XP032730242 und im Dokument US 2017/171178 A1 (REYNDERS TIM [US]) 15. Juni 2017 gezeigt.

Bei medizinischen Anwendungen kann es jedoch in Einzelfällen notwendig sein, die Pseudonymisierung der Daten wieder rückgängig zu machen, zum Beispiel beim Auftreten extremer Messwerte die eine Analyse der Ursachen erforderlich machen. Diese Re-Identifikation darf jedoch ausschließlich in der Sphäre der datenerhebenden Stelle erfolgen, eine Zugriffsmöglichkeit durch den Anbieter der Cloud-Anwendung muss ausgeschlossen werden. Ein Beispiel dafür sind erhöhte Strahlungsdosiswerte bei bildgebenden Verfahren im Krankenhaus, deren Ursachen auch aus regulatorischen Gründen analysiert und dokumentiert werden müssen.

Dies kann durch typischerweise verwendete Schlüsselverteilmechanismen zum Verteilen von symmetrischen Schlüsseln für bekannte Verschlüsselungsverfahren gemäß dem Stand der Technik nicht effizient und komfortabel bewerkstelligt werden.

Es ist eine Aufgabe der vorliegenden Erfindung, ein alternatives, komfortableres Verfahren und ein entsprechendes System sowie eine medizintechnische Anlage anzugeben, mit dem die oben beschriebenen Nachteile vermieden werden. Insbesondere war es eine Aufgabe der vorliegenden Erfindung, ein Verfahren zur Verfügung zu stellen, welches erlaubt, einem Benutzer die patientenbezogenen Daten in einer verschlüsselten Form über einen Cloud-Dienst bereitzustellen.

Diese Aufgabe wird durch ein Verfahren gemäß Patentanspruch 1, ein System gemäß Patentanspruch 12 und eine medizintechnische Anlage gemäß Patentanspruch 13 gelöst.

Das erfindungsgemäße Verfahren und das erfindungsgemäße System dienen typischerweise zur sicheren Übertragung eines (insbesondere medizinischen) Datensatzes zwischen einem Server und einem Benutzergerät mittels eines Cloud-Dienstes. Selbstverständlich sind aber auch andere Anwendungen wie z.B. die Übermittlung anderer sicherheitskritische Datensätze möglich.

Cloud Computing, was im Deutschen so viel wie "Rechnerwolke" oder "Datenwolke" heißt, betrifft im Rahmen dieser Erfindung die Bereitstellung von Speicherplatz als Dienstleistung über ein Netzwerk, insbesondere über das Internet. Der Speicherplatz muss dabei nicht auf dem lokalen Rechnernetz einer medizinischen Einrichtung installiert sein, sondern kann nach Bedarf von einem oder mehreren Anbietern bezogen werden. Die Erfindung ist besonders vorteilhaft einsetzbar, wenn dieser Speicherplatz außerhalb der medizinischen Einrichtung liegt, in der sich Benutzergerät und Server befinden und nur über eine (naturgemäß als unsicher einzustufende) Internetverbindung erreichbar ist. Auch wenn die Bezeichnung "Cloud" aus dem Englischen stammt, ist sie für entsprechende Dienste auch in der deutschen Sprache gebräuchlich. Im Folgenden werden zum besseren Verständnis die Begriffe "Cloud" oder "Cloud-Dienst" für einen Dienst verwendet, der Speicherplatz zur Verfügung stellt und über den Daten auf diesem Speicherplatz abspeichert oder aus diesem abgerufen werden können.

Das erfindungsgemäße Verfahren zur sicheren Übertragung eines (insbesondere medizinischen) Datensatzes zwischen einem Server und einem Benutzergerät mittels eines Cloud-Dienstes umfasst die folgenden Schritte.

- Bereitstellung und/oder Erzeugung eines benutzerseitigen (ersten) Diffie Hellman Schlüsselpaares durch das Benutzergerät. Dieses benutzerseitige Diffie Hellman Schlüsselpaar umfasst naturgemäß einen geheimen Benutzerschlüssel und einen öffentlichen Benutzerschlüssel. Die Grundlagen der Diffie Hellman Verschlüsselung sind dem Fachmann bekannt und werden in Grundzügen im einleitenden Teil beschrieben. Bei einer bevorzugten Verschlüsselung wird ein Schlüsselpaar pro Anfrage, ein Schlüsselpaar pro Benutzer oder ein Schlüsselpaar pro angefragtem Server generiert bzw. verwendet. Der Begriff "Erstellen" bedeutet hier, dass ein Schlüsselpaar berechnet wird, ein "Bereitstellen" die Bereitstellung eines bereits berechneten Schlüsselpaares, z.B. aus einem Datenspeicher.
- Übermittlung des öffentlichen Benutzerschlüssels an den Server. Der öffentliche Benutzerschlüssel wird dabei über eine Datenleitung vom Benutzergerät an den Server gesendet. Da dies in der Regel innerhalb eines Netzwerks einer medizinischen Einrichtung geschieht, die nach außen hin oftmals datentechnisch gesichert ist, kann durchaus eine normale http-Verbindung verwendet werden (http: "Hypertext Transfer Protocol"; dt.: "Hypertext-Übertragungsprotokoll"). Typischerweise werden aber auch in diesem Umfeld zumindest serverseitig gesicherte Verbindungen mittels https genutzt. Hierbei authentisiert sich der Server gegenüber dem Benutzer.
- Bereitstellung und/oder Erzeugung eines serverseitigen (zweiten) Diffie Hellman Schlüsselpaares durch den Server. Dieses serverseitige Schlüsselpaar umfasst naturgemäß einen geheimen Serverschlüssel und einen öffentlichen Serverschlüssel. Je nach Anwendungsfall ist es bevorzugt, dass pro Datensatz und/oder pro Benutzergerät ein individuelles Schlüsselpaar aus öffentlichem Serverschlüssel und geheimen Serverschlüssel vom Server erzeugt wird. Es kann aber auch der Fall vorliegen, dass das Schlüsselpaar bereits vorher erzeugt wurde und in diesem Schritt lediglich durch Abrufen aus einem Speicher bereitgestellt wird.
- Bereitstellung eines Datensatzes auf dem Server. Dieser Datensatz umfasst typischerweise patientenbezogene Daten, insbesondere medizinische Bilder oder Messergebnisse, die vertraulich sind.

- Generierung eines serverseitigen Diffie Hellman Schlüssels auf dem Server unter Verwendung des geheimen Serverschlüssels und des öffentlichen Benutzerschlüssels und Verschlüsselung des Datensatzes auf dem Server mittels des aus dieser Schlüsselgenerierung resultierenden serverseitigen Diffie Hellman Schlüssels. Der Datensatz befindet sich zur Verschlüsselung bevorzugt auf dem Server und kann von diesem beispielsweise vor einem medizinischen Gerät oder aus einer Datenbank abgerufen worden sein. Der Datensatz kann sich aber auch seit seiner Erstellung auf dem Server befunden haben.
- Übermittlung des verschlüsselten Datensatzes (und ggf. des öffentlichen Diffie Hellman Serverschlüssels) an den Cloud-Dienst. Dies kann beispielsweise mittels bekannter Verfahren und Protokolle zur Übertragung von Daten in Netzwerken erfolgen. Da der Cloud-Dienst oftmals mittels des Internets erreichbar ist, kann als möglicher Übertragungsweg auch eine Internetleitung (mit den entsprechenden Protokollen) verwendet werden.
- Abrufen des öffentlichen Serverschlüssels und Abrufen des verschlüsselten Datensatzes von dem Cloud-Dienst mittels des Benutzergeräts. Hierbei wird selbstverständlich der öffentliche Serverschlüssel desjenigen Schlüsselpaares abgerufen, der zur Generierung des Verschlüsselungsschlüssels für den betreffenden verschlüsselten Datensatz genutzt wurde. Bevorzugt wurde dieser öffentlichen Serverschlüssel zusammen mit dem verschlüsselten Datensatz durch den Cloud-Dienst (in der Cloud) abgespeichert und es werden beide Komponenten vom Cloud-Dienst abgerufen, jedoch sind je nach Anwendungsfall auch andere Varianten denkbar. Dies wird weiter unten noch ausführlicher beschrieben.
- Generierung eines benutzerseitigen Diffie Hellman Schlüssels auf dem Benutzergerät unter Verwendung des geheimen Benutzerschlüssels und des abgerufenen öffentlichen Serverschlüssels und Entschlüsselung des verschlüsselten Datensatzes auf dem Benutzergerät mit dem aus dieser Schlüsselgenerierung resultierenden benutzerseitigen Diffie Hellman Schlüssel.

Es sollte dabei beachtet werden, dass der ursprüngliche Datensatz stets serverseitig verschlüsselt wird und der Benutzer mit seinem Benutzergerät stets den verschlüsselten Datensatz von der Cloud abruft. Der verschlüsselte Datensatz wird also weder durch das Benutzergerät vom Server direkt abgerufen noch wird der Datensatz vom Benutzergerät direkt verschlüsselt und an die Cloud gesendet. Im Rahmen der Erfindung ist eine Kommunikation ("Anfrage") zwischen Benutzergerät und Server nur erforderlich zur Übertragung des öffentlichen Benutzerschlüssels und ggf. zur Mitteilung, dass ein bestimmter Datensatz in der Cloud abgespeichert werden soll.

Zusätzlich folgt bevorzugt der Schritt:
Bereitstellen des (entschlüsselten) Datensatzes mittels des Benutzergeräts, wobei das Bereitstellen bevorzugt ein Anzeigen, Speichern und/oder Verarbeiten des Datensatzes umfasst.

Vorsorglich wird darauf hingewiesen, dass die Reihenfolge der Verfahrensschritte nicht auf die dargestellte festgelegt ist, sofern nicht diesbezüglich technische Gründe eine Reihenfolge vorgeben. So kann z.B. die Bereitstellung des Datensatzes bereits vor der Generierung eines oder beider Schlüsselpaare erfolgen, ebenso kann z.B. das serverseitige Schlüsselpaar auch vor dem benutzerseitigen Schlüsselpaar erzeugt werden. Im Grunde ist nur wichtig, dass zu einem Schritt die dafür relevanten Komponenten vorliegen.

Im Unterschied zum Stand der Technik wird die an sich zwischen zwei Teilnehmern bekannte Diffie-Hellman-Verschlüsselung zu einer 3-Punkt-Kommunikation abgewandelt, in dem eine Diffie Hellman Schlüsselaushandlung so adaptiert wird, dass der resultierende Schlüssel, der für die Verschlüsselung genutzt wurde, zu einem späteren Zeitpunkt wiederhergestellt werden kann.

Ein Vorteil der Erfindung ist, dass zusätzlich zu den verschlüsselten Daten Informationen zu deren Entschlüsselung bereitgestellt werden, ohne dass der Cloud-Dienst mittels dieser Informationen in der Lage ist, die Daten selbst zu entschlüsseln.

Die Erfindung erlaubt auf vorteilhafte Weise aus verschiedenen Netzen, also von verschiedenen Benutzergeräten (Clients) auf die Daten in der Cloud zuzugreifen, ohne dass der Server für alle Clients zugreifbar sein muss. Des Weiteren vereinfacht das erfindungsgemäße Prinzip die Infrastruktur. Mit dem vorgeschlagenen Ansatz kann auf der Serverseite ein einfacheres Management von Zertifikaten für die Authentisierung im Rahmen der https Kommunikation erfolgen, da die Sicherheit der Daten im Cloud Dienst im Wesentlichen über die Diffie Hellman Schlüsselgenerierung sichergestellt wird.

Das erfindungsgemäße System zur sicheren Übertragung eines (insbesondere medizinischen) Datensatzes zwischen einem Server und einem Benutzergerät mittels eines Cloud-Dienstes umfasst einen Server, ein Benutzergerät und eine Datenverbindung zwischen Server und Benutzergerät und eine Datenschnittstelle für eine Datenverbindung zwischen Server und Benutzergerät mit einem Cloud-Dienst. Der Server und das Benutzergerät sind dabei besonders konfiguriert, wie im Folgenden genauer ausgeführt wird.
- Das Benutzergerät ist zum Abrufen und/oder zur Erzeugung eines benutzerseitigen Diffie Hellman Schlüsselpaares umfassend einen geheimen Benutzerschlüssel und einen öffentlichen Benutzerschlüssel ausgelegt.
- Das System ist zur Übermittlung des öffentlichen Benutzerschlüssels an den Server ausgelegt. Dazu wird die Datenverbindung zwischen Server und Benutzergerät verwendet.
- Der Server ist zum Abrufen und/oder zur Erzeugung eines serverseitigen Diffie Hellman Schlüsselpaares umfassend einen geheimen Serverschlüssel und einen öffentlichen Serverschlüssel ausgelegt. Wie oben bereits angesprochen worden ist, kann das serverseitige Diffie Hellman Schlüsselpaar aus einem Speicher des Servers oder sogar von einer Datenbank bezogen werden. Diese Verbindung zur Datenbank sollte sicher sein bzw. das serverseitige Diffie Hellman Schlüsselpaar verschlüsselt übertragen werden. Besonders bevorzugt ist, wenn der Server dazu ausgestaltet ist sowohl ein Diffie Hellman Schlüsselpaar zu erzeugen als auch ein Diffie Hellman Schlüsselpaar (aus einer Speichereinheit) abzurufen.
- Der Server ist zum Abrufen eines Datensatzes ausgelegt. Dieser Datensatz kann aus einem Speicher des Servers oder sogar von einer Datenbank bezogen werden. Diese Verbindung zur Datenbank sollte sicher sein bzw. der Datensatz verschlüsselt übertragen werden. Der Server kann sich in einem medizintechnischen Gerät (einer medizintechnischen Anlage) befinden und mit diesem Gerät ermittelte medizintechnische Daten, z.B. Messergebnisse oder Bilder, direkt nach dem erfindungsgemäßen Prinzip (mit dem erfindungsgemäßen Verfahren) verschlüsselt werden.
- Der Server ist zu einer Generierung eines serverseitigen Diffie Hellman Schlüssels unter Verwendung des geheimen Serverschlüssels und des öffentlichen Benutzerschlüssels und zur Verschlüsselung des Datensatzes mittels des resultierenden serverseitigen Diffie Hellman Schlüssels ausgelegt.
- Der Server ist zur Übermittlung des verschlüsselten Datensatzes an den Cloud-Dienst ausgelegt.
- Das Benutzergerät ist zum Abrufen des verschlüsselten Datensatzes und des öffentlichen Serverschlüssels von dem Cloud-Dienst ausgelegt.
- Das Benutzergerät ist zur Generierung eines benutzerseitigen Diffie Hellman Schlüssels unter Verwendung des geheimen Benutzerschlüssels und des abgerufenen öffentlichen Serverschlüssels und zur Entschlüsselung des verschlüsselten Datensatzes auf dem Benutzergerät mit dem resultierenden benutzerseitigen Diffie Hellman Schlüssel ausgelegt.

Bevorzugt ist das Benutzergerät zusätzlich zum Bereitstellen des (entschlüsselten) Datensatzes ausgelegt, wobei das Bereitstellen bevorzugt ein Anzeigen, Speichern und/oder Verarbeiten des Datensatzes umfasst.

Es sollte beachtet werden, dass die vorgenannten und die im folgenden beschriebenen Systemkomponenten (einzelne Systemkomponenten oder jede der Systemkomponenten, also Server bzw. Benutzergerät bzw. Datenbank) eine oder mehrere der folgende Teilkomponenten aufweisen können, wobei jede dieser Teilkomponenten wiederum mehrere zusammenwirkende Teilkomponenten umfassen kann:
- eine Recheneinheit ("CPU"), insbesondere eine Recheneinheit der Gruppe Prozessor, Mikroprozessor, integrierter Schaltkreis, GPU, ASIC, FPGA,
- eine Speichereinheit, insbesondere eine Speichereinheit der Gruppe Magnetspeicher (z.B. eine Festplatte), Halbleiterspeicher (z.B. eine Solid State Disk), optische Speicher (z.B. DVD) oder Mischformen dieser Datenspeicher,
- eine Schnittstelle (z.B. ein Datenbus, eine WLAN, eine LAN oder eine USB-Schnittstelle).

Eine erfindungsgemäße medizintechnische Anlage ist bevorzugt zur Aufnahme und/oder zur Bearbeitung medizintechnischer Bilder gestaltet und zur Integration als Server in ein Verfahren nach einem der Ansprüche 1 bis 11 ausgelegt. Alternativ oder ergänzend umfasst die medizintechnische Anlage einen Server von einem erfindungsgemäßen System. Die medizintechnische Anlage umfasst also einen Server ausgelegt:
- zum Empfang eines öffentlichen Benutzerschlüssels von einem Benutzersystem,
- zum Abrufen und/oder zur Erzeugung eines serverseitigen Diffie Hellman Schlüsselpaares umfassend einen geheimen Serverschlüssel und einen öffentlichen Serverschlüssel,
- zum Abrufen eines Datensatzes,
- zu einer Generierung eines serverseitigen Diffie Hellman Schlüssels unter Verwendung des geheimen Serverschlüssels und des öffentlichen Benutzerschlüssels und zur Verschlüsselung des Datensatzes mittels des resultierenden serverseitigen Diffie Hellman Schlüssels, und
- zur Übermittlung des verschlüsselten Datensatzes an einen Cloud-Dienst.

Solch ein Server kann aber auch unabhängig von einer medizintechnischen Anlage vorliegen. Ein erfindungsgemäßer Server, welcher im Rahmen des erfindungsgemäßen Verfahrens zur sicheren Übertragung eines Datensatzes eingesetzt werden kann, umfasst zumindest:
- Eine Empfangseinheit, ausgelegt zum Empfang eines öffentlichen Benutzerschlüssels von einem Benutzersystem,
- eine Schlüsseleinheit, ausgelegt zum Abrufen und/oder zur Erzeugung eines serverseitigen Diffie Hellman Schlüsselpaares umfassend einen geheimen Serverschlüssel und einen öffentlichen Serverschlüssel,
- eine Datenschnittstelle, ausgelegt zum Abrufen eines Datensatzes,
- eine Verschlüsselungseinheit, ausgelegt zu einer Generierung eines serverseitigen Diffie Hellman Schlüssels unter Verwendung des geheimen Serverschlüssels und des öffentlichen Benutzerschlüssels und zur Verschlüsselung des Datensatzes mittels des resultierenden serverseitigen Diffie Hellman Schlüssels, und
- eine Übermittlungseinheit, ausgelegt zur Übermittlung des verschlüsselten Datensatzes an einen Cloud-Dienst.

Ein erfindungsgemäßer Server ist also in der Lage, ein erfindungsgemäßes (serverseitiges) Verfahren durchzuführen, welches zumindest die folgenden Schritte umfasst:
- Empfang eines öffentlichen Benutzerschlüssels von einem Benutzersystem,
- Abrufen und/oder Erzeugung eines serverseitigen Diffie Hellman Schlüsselpaares umfassend einen geheimen Serverschlüssel und einen öffentlichen Serverschlüssel,
- Abrufen eines Datensatzes,
- Generierung eines serverseitigen Diffie Hellman Schlüssels unter Verwendung des geheimen Serverschlüssels und des öffentlichen Benutzerschlüssels und zur Verschlüsselung des Datensatzes mittels des resultierenden serverseitigen Diffie Hellman Schlüssels, und
- Übermittlung des verschlüsselten Datensatzes an einen Cloud-Dienst.

Ein erfindungsgemäßes Benutzergerät, welches im Rahmen des erfindungsgemäßen Verfahrens zur sicheren Übertragung eines Datensatzes eingesetzt werden kann, umfasst zumindest:
- eine Datenschnittstelle, ausgelegt für eine Datenverbindung zu einem Server,
- eine Datenschnittstelle, ausgelegt für eine Datenverbindung mit einem Cloud-Dienst,
- eine Schlüsseleinheit, ausgelegt zum Abrufen und/oder zur Erzeugung eines benutzerseitigen Diffie Hellman Schlüsselpaares umfassend einen geheimen Benutzerschlüssel und einen öffentlichen Benutzerschlüssel,
- eine Sendeeinheit, ausgelegt zur Übermittlung des öffentlichen Benutzerschlüssels an einen Server,
- eine Abrufungseinheit, ausgelegt zum Abrufen des öffentlichen Serverschlüssels und zum Abrufen eines verschlüsselten Datensatzes von einem Cloud-Dienst,
- eine Entschlüsselungseinheit, ausgelegt zu einer Generierung eines benutzerseitigen Diffie Hellman Schlüssels unter Verwendung des geheimen Benutzerschlüssels und eines abgerufenen öffentlichen Serverschlüssels und zur Entschlüsselung des verschlüsselten Datensatzes auf dem Benutzergerät mit dem resultierenden benutzerseitigen Diffie Hellman Schlüssel.

Ein erfindungsgemäßes Benutzersystem ist also in der Lage, ein erfindungsgemäßes (benutzerseitiges) Verfahren durchzuführen, welches zumindest die folgenden Schritte umfasst:
- Abrufen und/oder zur Erzeugung eines benutzerseitigen Diffie Hellman Schlüsselpaares umfassend einen geheimen Benutzerschlüssel und einen öffentlichen Benutzerschlüssel,
- Übermittlung des öffentlichen Benutzerschlüssels an einen Server,
- Abrufen des öffentlichen Serverschlüssels und Abrufen eines verschlüsselten Datensatzes von einem Cloud-Dienst,
- Generierung eines benutzerseitigen Diffie Hellman Schlüssels unter Verwendung des geheimen Benutzerschlüssels und eines abgerufenen öffentlichen Serverschlüssels und Entschlüsselung des verschlüsselten Datensatzes auf dem Benutzergerät mit dem resultierenden benutzerseitigen Diffie Hellman Schlüssel.

Ein erfindungsgemäßes System umfasst bevorzugt zumindest ein erfindungsgemäßes Benutzergerät und einen erfindungsgemäßen Server.

Ein Großteil der zuvor genannten Komponenten des Systems, können ganz oder teilweise in Form von Softwaremodulen in einem Prozessor einer entsprechenden medizintechnischen Anlage realisiert werden. Eine weitgehend softwaremäßige Realisierung hat den Vorteil, dass auch schon bisher verwendete medizintechnische Anlagen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem bzw. eine medizintechnische Anlage ladbar ist, mit Programmabschnitten, um alle Schritte des erfindungsgemäßen Verfahrens auszuführen, wenn das Programm in dem Rechensystem bzw. der medizintechnischen Anlage ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Rechensystem bzw. zur medizintechnischen Anlage und/oder zur Speicherung an oder in dem Rechensystem bzw. der medizintechnischen Anlage kann ein computerlesbares Medium, beispielsweise ein Memorystick, eine Festplatte oder ein sonstiger transportabler oder fest eingebauter Datenträger dienen, auf welchem die von einem Rechensystem bzw. einer Rechnereinheit der medizintechnischen Anlage einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Es sollte beachtet werden, dass der Benutzer seinen geheimen Benutzerschlüssel für die Entschlüsselung des Datensatzes abspeichern und vorrätig halten sollte, da er ansonsten nicht mehr in der Lage wäre, basierend auf dem öffentlichen Serverschlüssel den Schlüssel zur Entschlüsselung des Datensatzes zu rekonstruieren. Wenn ein Benutzer für jeden Verschlüsselungsvorgang ein neues Schlüsselpaar generiert, muss er folglich auch alle geheimen Benutzerschlüssel vorhalten. In diesem Fall ist es jedoch von Vorteil, wenn er einen Hinweis erhält, welchen verschlüsselten Datensatz in der Cloud mit welchem geheimen Benutzerschlüssel entschlüsselt werden kann. Beispielsweise kann der Datensatz einen entsprechenden Hinweis enthalten, welcher geheime Benutzerschlüssel verwendet werden muss (ohne diesen jedoch zu offenbaren). Diese Problematik wird weiter unten ausführlicher behandelt.

Gemäß einem bevorzugten Verfahren wird der verschlüsselte Datensatz zusammen mit dem öffentlichen Serverschlüssel an die Cloud übermittelt. Nach der Übermittlung ist bevorzugt, dass der verschlüsselte Datensatz und der öffentliche Serverschlüssel in der Cloud zusammen abgespeichert werden. Ebenso ist bevorzugt, dass der verschlüsselte Datensatz und der öffentliche Serverschlüssel in der Cloud mit einer Referenz aufeinander abgespeichert werden. Dies hat den Vorteil, dass der verschlüsselte Datensatz und der öffentliche Serverschlüssel bei einem Abruf durch das Benutzergerät gemeinsam an dieses gesendet werden kann.

Theoretisch ist es möglich, dass der öffentliche Serverschlüssel nicht an die Cloud gesendet wird, sondern z.B. vom Server bezogen werden kann. In diesem Fall muss aber dem Benutzergerät bekannt sein, mit welchem öffentlichen Serverschlüssel der verschlüsselte Datensatz verschlüsselt worden ist, so dass es auch in diesem Fall auch von Vorteil ist, wenn der verschlüsselte Datensatz und der öffentliche Serverschlüssel mit einer Referenz aufeinander abgespeichert werden. Alternativ kann aber auch der Server anhand des abfragenden Benutzers eine Zuordnung zu einem verschlüsselten Datensatz realisieren.

Es ist je nach Anwendung bevorzugt, dass der verschlüsselte Datensatz zusammen mit dem öffentlichen Serverschlüssel durch den Server digital signiert wird. Dies bindet dann den öffentlichen Serverschlüssel an den Datensatz.

Gemäß einem bevorzugten Verfahren erfolgt zusätzlich zur Übermittlung des öffentlichen Benutzerschlüssels auch eine Übermittlung einer Benutzer-Referenznummer durch das Benutzergerät an den Server. Diese Benutzer-Referenznummer ist dabei so beschaffen, dass sie Rückschlüsse auf den Benutzer und/oder das Benutzergerät zulässt. Dies ermöglicht bei Vorliegen einer großen Anzahl von verschlüsselten Datensätzen im Cloud-Dienst eine sehr gute Zuordnung zu den Benutzern bzw. Benutzergeräten, die Zugriff auf diese Datensätze haben sollen und dient insbesondere einer effektiven Suche.

Es ist bevorzugt, dass im Rahmen der Übermittlung des verschlüsselten Datensatzes an den Cloud-Dienst (durch den Server) zusätzlich die Benutzer-Referenznummer übermittelt wird. Nach der Übermittlung ist bevorzugt, dass der verschlüsselte Datensatz und die Benutzer-Referenznummer in der Cloud zusammen abgespeichert werden. Ebenso ist bevorzugt, dass der verschlüsselte Datensatz und die Benutzer-Referenznummer in der Cloud mit einer Referenz aufeinander abgespeichert werden. Dies hat den Vorteil, dass der verschlüsselte Datensatz und die Benutzer-Referenznummer miteinander verbunden sind und bei einem Abruf oder einer Suchanfrage durch ein Benutzergerät eine Zugehörigkeit geprüft werden kann.

Die Benutzer-Referenznummer ist in erster Linie für die Assoziation der (verschlüsselten) Datensätze mit einem Benutzer bzw. Benutzergerät von Vorteil. Wenn ein Server verschlüsselte Datensätze ohne Benutzer-Referenznummer an den Cloud-Dienst sendet, ist es für diesen schwieriger eine Anfrage eines Benutzergeräts mit den entsprechenden verschlüsselten Datensätzen zu beantworten.

Zu der oben angesprochenen Zuordnung eines geheimen Benutzerschlüssels zu einem abgerufenen verschlüsselten Datensatz kann die Benutzer-Referenznummer vorteilhaft genutzt werden. Der Benutzer kann diese verwenden, um die entsprechende Anfrage für einen Datensatz damit zu verbinden. Es wird bevorzugt dazu (z.B. pro Datensatz) ein Benutzerschlüsselpaar (benutzerseitiges Diffie Hellman Schlüsselpaar) generiert, welches datentechnisch mit der Benutzer-Referenznummer verknüpft wird (zumindest der geheime Benutzerschlüssel) und dann der öffentliche Benutzerschlüssel zusammen mit der Benutzer-Referenznummer an den Server gesandt und von diesem der verschlüsselte Datensatz zusammen mit der Benutzer-Referenznummer in der Cloud abgespeichert wird. Beim Abruf steht die Benutzer-Referenznummer dem Benutzergerät zur Verfügung und der korrekte geheime Benutzerschlüssel kann verwendet werden.

Alternativ kann der Benutzer nur die Anfrage über die Benutzer-Referenznummer referenzieren und ein spezifisches Benutzerschlüsselpaar für alle Anfragen verwenden. Eine weitere Alternative wäre der Fall, dass pro anzufragenden Server ein Benutzerschlüsselpaar verwendet wird. Damit könnten dann alle Datenabfragen mit einem serverspezifischen Benutzerschlüssel verbunden werden, wobei in diesem Fall zusammen mit dem verschlüsselten Datensatz in der Cloud ein Hinweis vorhanden ist, welcher Server diesen verschlüsselt hat.

Die Verwaltung aller drei Alternativen liegt auf der Seite des Benutzers und kann von diesem bevorzugt ausgewählt werden. Es ist auch bevorzugt, dass die URL zum verschlüsselten Datensatz zumindest einen Teil der Benutzer-Referenznummer umfasst.

Gemäß einem bevorzugten Verfahren wird vom Server ein vorher erstelltes Schlüsselpaar für eine definierte Anzahl von Datensätzen und/oder für eine Gruppe von Benutzergeräten verwendet.

Theoretisch kann dies ein einziges serverseitiges Schlüsselpaar für alle Vorgänge sein, von dem dann der öffentliche Serverschlüssel direkt an die Benutzergeräte übermittelt werden kann. Selbstverständlich ist diese Alternative mit einer gewissen Beeinträchtigung der Sicherheit verbunden. Sicherer ist es, wenn für jedes Benutzergerät ein unterschiedliches Schlüsselpaar generiert wird. Auch hier kann theoretisch ein einziges serverseitiges Schlüsselpaar für jeweils ein Benutzergerät verwendet werden und der öffentliche Serverschlüssel vom Server zu diesem Benutzergerät gesendet und dort abgespeichert werden. In diesem Falle wäre eine Benutzer-Referenznummer für die verschlüsselten Datensätze sehr vorteilhaft. In dem Fall, in dem der öffentliche Serverschlüssel auf anderem Wege als von dem Cloud-Dienst zum Benutzersystem gelangt, ist eine Referenz sehr vorteilhaft oder gar notwendig, die angibt, welcher öffentliche Schlüssel zur Entschlüsselung welches Datensatzes benötigt wird.

Gemäß einem bevorzugten Verfahren wird im Rahmen des Abrufens des verschlüsselten Datensatzes dieser zusammen mit dem öffentlichen Serverschlüssel durch das Benutzergerät vom Cloud-Dienst abgerufen, bevorzugt unter Angabe der Benutzer-Referenznummer. Dies ist besonders vorteilhaft, wenn nicht ein (benutzerseitiges) Diffie Hellman Schlüsselpaar für alle oder für jeweils ein Benutzergerät generiert wird, sondern ein Schlüsselpaar in Abhängigkeit von den Datensätzen (z.B. für jeden Datensatz neu) generiert wird. In diesem Fall ist insbesondere die Verknüpfung eines Datensatzes mit einer Benutzer-Referenznummer von Vorteil.

Der öffentliche Schlüssel des Servers wird vom Benutzergerät benötigt, um einen verschlüsselten Datensatz zu entschlüsseln. Eine Gruppe aus einem Datensatz zusammen mit dem öffentlichen Serverschlüssels (desjenigen Schlüsselpaares, mit dem der Schlüssel zur Verschlüsselung des Datensatz generiert worden ist), vereinfacht die Handhabung für das Benutzergerät.

Gemäß einem bevorzugten Verfahren führt nach der Übermittlung des öffentlichen Benutzerschlüssels an den Server dieser Server eine Authentisierung des Benutzergeräts durch. Bei erfolgreicher Authentisierung wird dann mit der Bereitstellung oder Erzeugung eines (serverseitigen) Diffie Hellman Schlüsselpaares durch den Server fortgefahren. Bevorzugt wird zusätzlich auch eine Autorisierung des Benutzergeräts geprüft.

Die Authentisierung erfolgt bevorzugt über ein Challenge Response Verfahren (dt.: "Aufforderung-Antwort-Verfahren"). Dies ist ein Authentifizierungsverfahren eines Teilnehmers auf Basis von Wissen. Hierbei stellt ein Teilnehmer eine Aufgabe (engl. challenge), die der andere lösen muss (engl. response), um zu beweisen, dass er eine bestimmte Information (Shared Secret) kennt, ohne diese Information selber zu übertragen.

Gemäß einem bevorzugten Verfahren wird vor dem Verschlüsseln des Datensatzes durch den Server eine Schlüsselableitung über eine Schlüsselableitungsfunktion durchgeführt. Dabei gehen bevorzugt in die Schlüsselableitungsfunktion Informationen und/oder Charakteristika der Datensatzbezeichnung ein, bevorzugt ein Datum (z.B. bei Magnetresonanztomographiebildern ein MRT-Datum. Die Schlüsselableitung hat den Vorteil, dass jeder Datensatz mit einem separaten abgeleiteten Schlüssel von einem Diffie Hellman Schlüssel verschlüsselt werden kann.

Bevorzugt erfolgt die Schlüsselableitung nach einem Verfahren der Gruppe PBKDF (Password-Based Key Derivation Function), HKDF (hash key derivation function), HMAC-SHA256 (hash-based message authentication code based on Secure Hash Algorithm 256) .

Gemäß einem bevorzugten Verfahren wird zur Übertragung des verschlüsselten Datensatzes und bevorzugt auch des öffentlichen Serverschlüssels und/oder der Benutzer-Referenznummer die Datenverbindung zwischen dem Server und dem Cloud-Dienst vom Server initiiert und der Cloud-Dienst sich mittels eines Zertifikats authentisiert. Dabei ist bevorzugt, dass sich auch der Server mit einem individuellen Berechtigungsnachweis authentisiert. Ein solcher Berechtigungsnachweis ist typischerweise eine Kombination aus einem Benutzernamen und einem Passwort oder aber ein Zertifikat mit zugehörigem privatem Schlüssel.

Gemäß einem bevorzugten Verfahren erfolgt die Übermittlung des verschlüsselten Datensatzes an den Cloud-Dienst und/oder das Abrufen des verschlüsselten Datensatzes von dem Cloud-Dienst über eine http-Verbindung. Zwischen Cloud und Server und/oder zwischen Cloud und Benutzergerät ist dabei eine https-Verbindung, also eine verschlüsselte http-Verbindung, bevorzugt (https: "Hypertext Transfer Protocol Secure"; dt.: "sicheres Hypertext-Übertragungsprotokoll"). Theoretisch könnte auf die Verschlüsselung einer Verbindung verzichtet werden und einfach eine http-Verbindung verwendet werden, da der übertragene Datensatz ja bereits verschlüsselt ist. Allerdings findet im Rahmen des https-Verfahrens automatisch eine Authentisierung der kommunizierenden Komponenten statt. Ohne diese wären Man-in-the-Middle Angriffe möglich, so dass ein Dritter die Daten verändern könnte. Insbesondere, könnte ein Angreifer die Assoziation Benutzer-Referenznummer und Datensatz verändern und so die Daten bestimmter Benutzer vertauschen. Daher ist eine https-Verbindung sehr vorteilhaft.

Gemäß einem bevorzugten Verfahren stellt der Cloud-Dienst dem Benutzergerät eine Publish/Subscribe-Schnittstelle zur Verfügung. Das Benutzergerät subskribiert bevorzugt dem Cloud-Dienst bezüglich des gewünschten verschlüsselten Datensatzes mit der Benutzer-Referenznummer. In der Softwarearchitektur ist Publish-Subscribe ein Nachrichtenmuster, bei dem ein Absender von Nachrichten (als "Publisher" bezeichnet) die Nachrichten nicht so programmieren, dass sie direkt an bestimmte Empfänger (sogenannte Abonnenten) gesendet werden. Stattdessen werden veröffentlichte Nachrichten vom Publisher in Klassen kategorisiert, ohne zu wissen, welche Abonnenten vorhanden sind. In ähnlicher Weise bekunden Abonnenten Interesse an einer oder mehreren Klassen und erhalten nur Nachrichten, die von Interesse sind, ohne notwendigerweise zu wissen, welche Publisher vorhanden sind.

Gemäß einem bevorzugten Verfahren wird das Benutzergerät informiert, sobald der Server den entsprechenden Datensatz an den Cloud-Dienst übermittelt hat.

Auch wenn auf den ersten Blick eine Benachrichtigung des Benutzers nicht notwendig erscheint, da dieser die Übersendung des Datensatzes an die Cloud mit der Übersendung seines öffentlichen Benutzerschlüssels initiiert hat, ist sie dennoch vorteilhaft. Beispielsweise kann der Fall vorliegen, dass der Benutzer mehrere Datensätze über den Server zu verschiedenen Zeiten beantragt hat und die Bereitstellung der Daten asynchron zur Anfrage erfolgt. Der Benutzer kann dann informiert werden, wenn einer der angefragten Datensätze verfügbar ist.

Mittels der Erfindung können auf vorteilhafte Weise personenbezogene sensitive Daten über Cloud Dienste zur Verfügung gestellt werden. Die Besonderheit dabei ist, dass auf der Benutzerseite keine eigenen Zertifikate bzw. keine öffentlich auflösbaren Zertifikate für die Authentisierung genutzt werden müssen. Dies erspart dem Benutzer die Konfiguration des entsprechenden Schlüsselmaterials und auch das laufende Management (Erneuerung, etc.). Insbesondere können Fehler vermieden werden, wenn ein Benutzer statt öffentlich auflösbarer Zertifikate (von einer öffentlichen Zertifizierungsstelle) selbst generierte Zertifikate verwenden möchte, um Kosten zu sparen. In diesem Fall muss das Benutzergerät zuerst ein Zertifikat akzeptieren das nicht überprüft werden kann. Dieser Vorgang kann ein Sicherheitsrisiko darstellen. Eine besonderer Vorteil der Erfindung ist, dass die zur Entschlüsselung notwendigen Informationen nicht von einer Public Key Infrastruktur (PKI) bereitgestellt werden müssen. Dadurch wird eine zusätzliche Konfiguration von Benutzergerät bzw. Server vermieden.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 eine einen Ablaufplan für einen möglichen Ablauf eines erfindungsgemäßen Verfahrens,
Figur 2 ein schematisches Ausführungsbeispiel eines erfindungsgemäßen Systems,
Figur 3 eine einen detaillierteren Ablaufplan für einen möglichen Ablauf eines erfindungsgemäßen Verfahrens,
Figur 4 eine grob schematische Darstellung einer bevorzugten medizintechnischen Anlage.

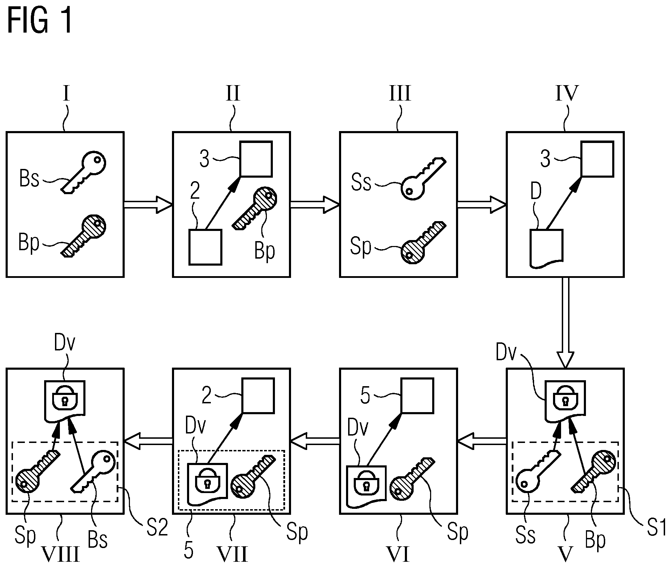

Figur 1 zeigt eine einen Ablaufplan für einen möglichen Ablauf eines erfindungsgemäßen Verfahrens zur sicheren Übertragung eines (insbesondere medizinischen) Datensatzes D zwischen einem Server 3 und einem Benutzergerät 2 mittels eines Cloud-Dienstes 5. Das Verfahren umfasst die folgenden Schritte:
In Schritt I erfolgt eine Bereitstellung oder Erzeugung eines benutzerseitigen Diffie Hellman Schlüsselpaares Bp, Bs durch das Benutzergerät 2, umfassend einen geheimen Benutzerschlüssel Bs und einen öffentlichen Benutzerschlüssel Bp.

In Schritt II erfolgt eine Übermittlung des öffentlichen Benutzerschlüssels Bp vom Benutzergerät 2 an den Server 3.

In Schritt III erfolgt eine Bereitstellung und/oder Erzeugung eines serverseitigen Diffie Hellman Schlüsselpaares Sp, Ss durch den Server 3, umfassend einen geheimen Serverschlüssel Ss und einen öffentlichen Serverschlüssel Sp.

In Schritt IV erfolgt eine Bereitstellung eines Datensatzes D auf dem Server 3. Dies ist beispielsweise eine Bilddatei.

In Schritt V erfolgt eine Generierung eines serverseitigen Diffie Hellman Schlüssels S1 auf dem Server 3 unter Verwendung des geheimen Serverschlüssels Ss und des öffentlichen Benutzerschlüssels Bp und Verschlüsselung des Datensatzes D auf dem Server 3 mittels des aus dieser Schlüsselgenerierung resultierenden serverseitigen Diffie Hellman Schlüssels S1.

In Schritt VI erfolgt eine Übermittlung des verschlüsselten Datensatzes Dv an den Cloud-Dienst 5. In diesem Beispiel wird zudem der öffentliche Serverschlüssel Sp verknüpft mit dem verschlüsselten Datensatz Dv an den Cloud-Dienst 5 gesendet und dort zusammen abgespeichert.

In Schritt VII erfolgt das Abrufen des öffentlichen Serverschlüssels Sp und Abrufen des verschlüsselten Datensatzes Dv von dem Cloud-Dienst 5 mittels des Benutzergeräts 2.

In Schritt VIII erfolgt eine Generierung eines benutzerseitigen Diffie Hellman Schlüssels S2 auf dem Benutzergerät unter Verwendung des geheimen Benutzerschlüssels Bs und des abgerufenen öffentlichen Serverschlüssels Sp und Entschlüsselung des verschlüsselten Datensatzes Dv auf dem Benutzergerät 2 mit dem aus dieser Schlüsselgenerierung resultierenden benutzerseitigen Diffie Hellman Schlüssel S2.

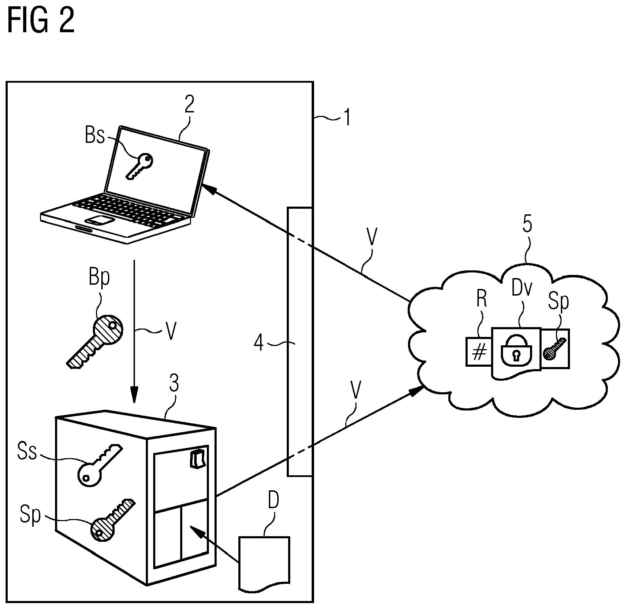

Figur 2 zeigt ein schematisches Ausführungsbeispiel eines erfindungsgemäßen Systems 1 zur sicheren Übertragung eines (insbesondere medizinischen) Datensatzes D zwischen einem Server 3 und einem Benutzergerät 2 mittels eines Cloud-Dienstes 5. Das System 1 umfasst einen Server 3, ein Benutzergerät 2 und eine Datenverbindung V zwischen Server 3 und Benutzergerät 2, sowie eine Datenschnittstelle 4 für eine Datenverbindung V zwischen Server 3 und Benutzergerät 2 mit einem Cloud-Dienst 5.

Das Benutzergerät 2 kann beispielsweise ein Rechner mit einem Netzwerkzugang und einem Browser sein. Der Server kann ein Webserver sein, der die gewünschten Daten bereitstellt. Das System 1 ist dabei Teil der Infrastruktur innerhalb eines Krankenhauses. Das Netzwerk des Krankenhauses ist in der Regel eingeschränkt und die drin betriebenen Komponenten (Benutzergerät 2 und Server 3) sind dann i.d.R. eingeschränkt bezüglich der Nutzung von https öffentlich auflösbaren Zertifikaten, da die Verteilung und Wartung von Zertifikaten und zugehörigen privaten Schlüsseln mit öffentlich auflösbaren Zertifikaten (von einer vertrauenswürdigen Zertifikatsstelle) nicht möglich ist. In dem dargestellten Beispiel wird weiterhin eine vertrauenswürdige Umgebung angenommen, so dass für die Datenverbindung V zwischen Benutzergerät 2 und Server 3 eine http-Verbindung verwendet werden kann. Alternativ kann hier jedoch ein eigenes Zertifikat installiert sein, das vom Benutzergerät als vertrauenswürdig konfiguriert ist und damit auch eine https Verbindung ermöglicht. Zum Cloud-Dienst kann dann eine Standard-https-Verbindung als Datenverbindung V verwendet werden.

Das Benutzergerät 2 ist zum Abrufen und/oder zur Erzeugung eines benutzerseitigen Diffie Hellman Schlüsselpaares Bp, Bs umfassend einen geheimen Benutzerschlüssel Bs und einen öffentlichen Benutzerschlüssel Bp ausgelegt.

Das System 1 ist zur Übermittlung des öffentlichen Benutzerschlüssels Bp an den Server 3 ausgelegt. Dargestellt ist der Fall, dass der öffentliche Benutzerschlüssel Bp gerade übermittelt wird. Zur besseren Übersicht ist der öffentliche Schlüssel schraffiert und der geheime Schlüssel weiß.

Der Server 3 ist zum Abrufen und/oder zur Erzeugung eines serverseitigen Diffie Hellman Schlüsselpaares Sp, Ss umfassend einen geheimen Serverschlüssel Ss und einen öffentlichen Serverschlüssel Sp ausgelegt. Diese werden zur besseren Unterscheidung zu den Benutzerschlüsseln Bp, Bs mit dem Bart nach oben dargestellt.

Der Server 3 ist zum Abrufen eines Datensatzes D ausgelegt.

Der Server 3 ist zudem zu einer Generierung eines serverseitigen Diffie Hellman Schlüssels S1 (s. Fig. 1) unter Verwendung des geheimen Serverschlüssels Ss und des öffentlichen Benutzerschlüssels Bp und zur Verschlüsselung des Datensatzes D mittels des daraus resultierenden serverseitigen Diffie Hellman Schlüssels S1 ausgelegt. Zur besseren Übersicht wird ein verschlüsselter Datensatz Dv mit einem Schloss dargestellt, wie in der Wolke des Cloud-Dienstes 5 zu sehen ist.

Der Server 3 ist zur Übermittlung des verschlüsselten Datensatzes Dv an den Cloud-Dienst 5 ausgelegt. Zudem ist in diesem Beispiel der Server 3 auch dazu ausgelegt, eine Benutzer-Referenznummer R an den Cloud-Dienst 5 zu senden. Dies ist hier bereits geschehen. In der Wolke des Cloud-Dienstes 5 ist der verschlüsselte Datensatz Dv verknüpft mit der Benutzer-Referenznummer R und dem öffentlichen Serverschlüssel Sp zu sehen. In diesem Beispiel wird immer derselbe öffentlichen Serverschlüssel Sp verwendet.

Das Benutzergerät 2 ist zum Abrufen des öffentlichen Serverschlüssels Sp und zum Abrufen des verschlüsselten Datensatzes Dv von dem Cloud-Dienst 5 ausgelegt.

Das Benutzergerät 2 ist auch zur Generierung eines benutzerseitigen Diffie Hellman Schlüssels S2 (s. Figur 1) unter Verwendung des geheimen Benutzerschlüssels Bs und des abgerufenen öffentlichen Serverschlüssels Sp und zur Entschlüsselung des verschlüsselten Datensatzes Dv auf dem Benutzergerät 2 mit dem daraus resultierenden benutzerseitigen Diffie Hellman Schlüssel S2 ausgelegt.

Bei einer Datenschnittstelle 4 kann es sich um eine Hardware- oder Softwareschnittstelle handeln (beispielsweise Ethernet, PCI-Bus, USB oder Firewire). Ein Benutzergerät 2 bzw. ein Server 3 kann Hardware-Elemente oder Software-Elemente aufweisen, beispielsweise eine CPU (englisches Akronym für "central processing unit"), eine GPU (englisches Akronym für "graphical processing unit"), einen Mikroprozessor, ein sogenanntes FPGA (englisches Akronym für "Field Programmable Gate Array") oder ein ASIC (englisches Akronym für "applicationspecific integrated circuit"). Eine Speichereinheit MU und/oder eine Trainingsspeichereinheit TMU kann als nicht dauerhafte Arbeitsspeicher (Random Access Memory, kurz RAM) oder als dauerhafter Massenspeicher (Festplatte, USB-Stick, SD-Karte, Solid State Disk) realisiert sein.

Die Datenschnittstelle 4 kann insbesondere mehrere Unterschnittstellen umfassen, die unterschiedliche Schritte der jeweiligen Verfahren ausführen. Mit anderen Worten kann die Datenschnittstelle 4 auch als Vielzahl von Datenschnittstellen 4 aufgefasst werden. Ein Benutzergerät 2 bzw. ein Server 3 kann insbesondere mehrere Unterrecheneinheiten umfassen, die unterschiedliche Schritte der jeweiligen Verfahren ausführen. Mit anderen Worten kann die Benutzergerät 2 und/oder der Server 3 auch als Vielzahl von diesbezüglichen Recheneinheiten aufgefasst werden.

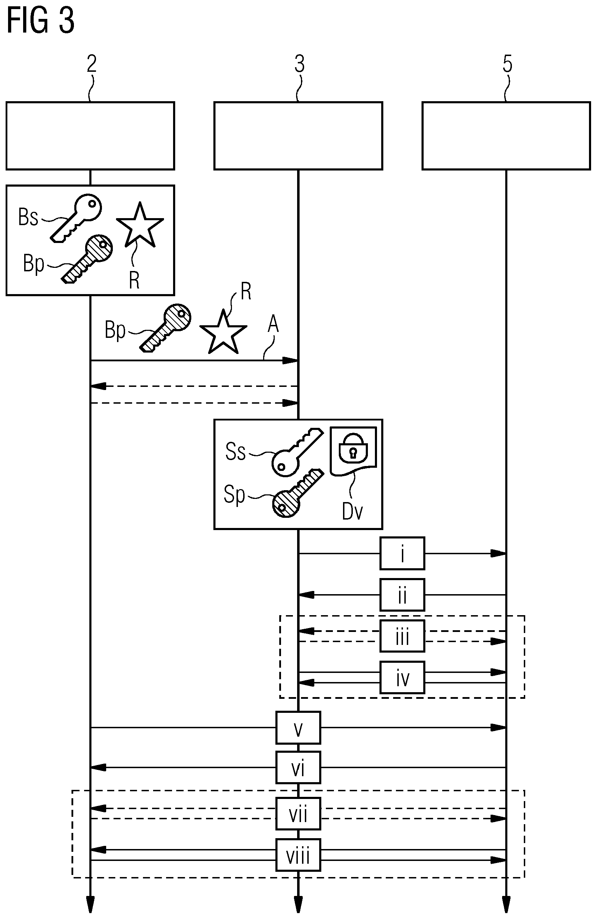

Figur 3 zeigt eine einen detaillierteren Ablaufplan für einen möglichen Ablauf eines erfindungsgemäßen Verfahrens.

Das Benutzergerät 2 möchte Zugriff auf einen bestimmten Datensatz D (der sich noch nicht in der Cloud befindet) und stellt dazu eine Anfrage A an den Server 3. Zuvor erzeugt es ein benutzerseitiges Diffie Hellman Schlüsselpaar bestehend aus dem öffentlichen Benutzerschlüssel Bp, dem geheimen Benutzerschlüssel Bs und einer Benutzer-Referenznummer R.

Der Server 3 empfängt die Anfrage und führt eine Authentisierung des Benutzergeräts 2 durch (gestrichelte Pfeile). Dies kann z.B. über ein standardmäßiges Challenge-Response Verfahren realisiert werden. Ist die Authentisierung und auch die Autorisierung des Benutzergeräts 2 erfolgreich, so erzeugt der Server 3 ein serverseitiges Diffie Hellman Schlüsselpaar bestehend aus dem öffentlichen Serverschlüssel Sp und dem geheimen Serverschlüssel Ss.

Der Server 3 ist nun in der Lage mittels des empfangenen öffentlichen Benutzerschlüssels Bp und dem geheimen Serverschlüssel Ss wie oben geschildert einen Schlüssel zu berechnen und einen verschlüsselten Datensatz Dv zu erstellen. In die Schlüsselableitungsfunktion können dabei Charakteristika der Datensatzbezeichnung eingehen. Diese Ableitung hat den Vorteil, dass jeder Datensatz mit einem separaten Schlüssel verschlüsselt werden kann.

Im Anschluss überträgt der Server 3 den verschlüsselten Datensatz Dv, zusammen mit seinem öffentlichen Serverschlüssel Sp und der Benutzer-Referenznummer R an den Cloud-Dienst 5. Die Transportverbindung zwischen dem netzinternen Server 3 und dem Cloud-Dienst 5 wird vom Server 3 initiiert (Pfeil 'i') und der Cloud-Dienst 5 authentisiert sich mittels eines Zertifikats (Pfeil 'ii'). Der Server wiederum authentisiert sich mit einem eigenen Credential (Pfeile iii), was typischerweise eine Kombination aus Username und Passwort darstellt. Danach erfolgt die Übermittlung des verschlüsselten Datensatzes Dv zusammen mit seinem öffentlichen Serverschlüssel Sp und der Benutzer-Referenznummer R durch den Server 3 gefolgt von einer Nachricht über den Erfolg seitens des Cloud-Dienstes 5 (Pfeile iv).

Bevorzugt ist eine Transport Layer Security Verbindung (TLS-Verbindung), die hier nach der Initiierung etabliert ist (gestrichelter Kasten).

Das Benutzergerät 2 kann nun eine Anfrage an den Cloud-Dienst 5 stellen und auf dieselbe Weise wie vorangehend geschildert eine TLS-Verbindung aufbauen (Pfeile v und vi, gestrichelter Kasten). Innerhalb dieser Verbindung kann das Benutzergerät 2 dann die Benutzer-Referenznummer R übermitteln. In einer alternativen Variante stellt der Cloud Dienst eine Publish-Subscribe Schnittstelle zur Verfügung wie mit dem Pfeilpaar vii angedeutet. In dieser Variante kann das Benutzergerät beim der Cloud-Dienst 5 auf das Event mit der Benutzer-Referenznummer R subskribieren. Der Cloud-Dienst 5 kann nun den bereitgestellten verschlüsselten Datensatz Dv zusammen mit dem öffentlichen Serverschlüssel Sp dem Benutzergerät 2 zur Verfügung stellen (Pfeilpaar viii).

Nur das Benutzergerät 2, das den geheimen Benutzerschlüssel Bs besitzt, ist nun in der Lage den passenden Schlüssel zu berechnen, um den verschlüsselten Datensatz Dv zu entschlüsseln.

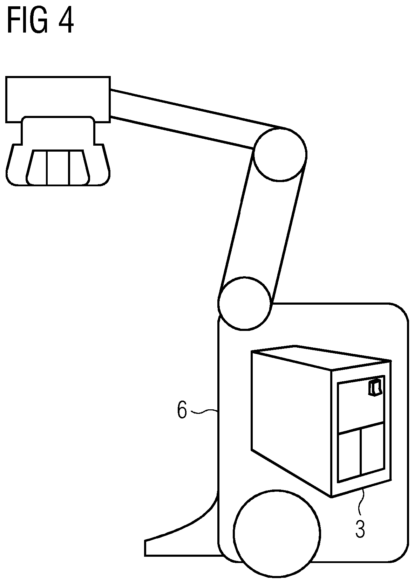

Figur 4 zeigt eine grob schematische Darstellung einer bevorzugten medizintechnischen Anlage 6. Diese umfasst einen Server 3 mit einer Funktionalität wie vorangehend beschrieben.

Es wird abschließend noch einmal darauf hingewiesen, dass es sich bei den vorhergehend detailliert beschriebenen Verfahren sowie bei dem dargestellten System 1 lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit", "Anlage" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur sicheren Übertragung eines Datensatzes (D) zwischen einem Server (3) und einem Benutzergerät (2) mittels eines Cloud-Dienstes (5) umfassend die Schritte:
- Bereitstellung oder Erzeugung (I) eines benutzerseitigen Diffie Hellman Schlüsselpaares (Bp, Bs) durch das Benutzergerät (2), umfassend einen geheimen Benutzerschlüssel (Bs) und einen öffentlichen Benutzerschlüssel (Bp),
- Übermittlung (II) des öffentlichen Benutzerschlüssels (Bp) an den Server (3),
- Bereitstellung und/oder Erzeugung (III) eines serverseitigen Diffie Hellman Schlüsselpaares (Sp, Ss) durch den Server (3), umfassend einen geheimen Serverschlüssel (Ss) und einen öffentlichen Serverschlüssel (Sp),
- Bereitstellung (IV) eines Datensatzes (D) auf dem Server (3),
- Generierung (V) eines serverseitigen Diffie Hellman Schlüssels (S1) auf dem Server (3) unter Verwendung des geheimen Serverschlüssels (Ss) und des öffentlichen Benutzerschlüssels (Bp) und Verschlüsselung des Datensatzes (D) auf dem Server (3) mittels des resultierenden serverseitigen Diffie Hellman Schlüssels (S1),
- Übermittlung (VI) des verschlüsselten Datensatzes (Dv) an den Cloud-Dienst (5),
- Abrufen (VII) des öffentlichen Serverschlüssels (Sp) und Abrufen des verschlüsselten Datensatzes (Dv) von dem Cloud-Dienst (5) mittels des Benutzergeräts (2),
- Generierung (VIII) eines benutzerseitigen Diffie Hellman Schlüssels (S2) auf dem Benutzergerät unter Verwendung des geheimen Benutzerschlüssels (Bs) und des abgerufenen öffentlichen Serverschlüssels (Sp) und Entschlüsselung des verschlüsselten Datensatzes (Dv) auf dem Benutzergerät (2) mit dem resultierenden benutzerseitigen Diffie Hellman Schlüssel (S2).

2. Verfahren nach Anspruch 1, wobei der verschlüsselte Datensatz (Dv) zusammen mit dem öffentlichen Serverschlüssel (Sp) an den Cloud-Dienst (5) übermittelt wird und
bevorzugt der verschlüsselte Datensatz (Dv) und der öffentliche Serverschlüssel (Sp) in dem Cloud-Dienst (5) zusammen oder zumindest mit einer Referenz aufeinander abgespeichert werden,
wobei bevorzugt der verschlüsselte Datensatz (Dv) zusammen mit dem öffentlichen Serverschlüssel (Sp) durch den Server (3) digital signiert wird.

3. Verfahren nach einem der vorangehenden Ansprüche, wobei zusätzlich zur Übermittlung des öffentlichen Benutzerschlüssels (Bp) auch eine Übermittlung einer Benutzer-Referenznummer (R) durch das Benutzergerät (2) an den Server (3) erfolgt,
wobei bevorzugt im Rahmen der Übermittlung des verschlüsselten Datensatzes (Dv) an den Cloud-Dienst (5) zusätzlich die Benutzer-Referenznummer (R) übermittelt wird und
bevorzugt die Benutzer-Referenznummer (R) und der verschlüsselte Datensatz (Dv) durch den Cloud-Dienst (5) zusammen oder zumindest mit einer Referenz aufeinander abgespeichert werden.

4. Verfahren nach einem der vorangehenden Ansprüche, wobei vom Server (3) ein vorher erstelltes serverseitiges Diffie-Hellman Schlüsselpaar (Ss, Sp) für eine definierte Anzahl von Datensätzen (D) und/oder für eine Gruppe von Benutzergeräten (2) verwendet wird.

5. Verfahren nach einem der vorangehenden Ansprüche, wobei im Rahmen des Abrufens des verschlüsselten Datensatzes (Dv) dieser zusammen mit dem öffentlichen Serverschlüssel (Sp) durch das Benutzergerät (2) vom Cloud-Dienst (5) abgerufen wird, bevorzugt unter Angabe der Benutzer-Referenznummer (R).

6. Verfahren nach einem der vorangehenden Ansprüche, wobei nach der Übermittlung des öffentlichen Benutzerschlüssels (Bp) an den Server (3) dieser eine Authentisierung des Benutzergeräts (2) durchführt, bevorzugt über ein Challenge Response Verfahren, und bei erfolgreicher Authentisierung mit der Bereitstellung oder Erzeugung eines serverseitigen Diffie Hellman Schlüsselpaares (Ss, Sp) durch den Server (3) fortgefahren wird,
wobei bevorzugt zusätzlich auch eine Autorisierung des Benutzergeräts (2) geprüft wird.

7. Verfahren nach einem der vorangehenden Ansprüche, wobei vor dem Verschlüsseln des Datensatzes (D) durch den Server (3) eine Schlüsselableitung über eine Schlüsselableitungsfunktion durchgeführt wird, bevorzugt nach einem Verfahren der Gruppe PBKDF, HKDF, HMAC-SHA256,
wobei bevorzugt in die Schlüsselableitungsfunktion Informationen und/oder Charakteristika der Datensatzbezeichnung eingehen.

8. Verfahren nach einem der vorangehenden Ansprüche, wobei zur Übertragung des verschlüsselten Datensatzes (Dv) und bevorzugt auch des öffentlichen Serverschlüssels (Sp) und/oder der Benutzer-Referenznummer (R) die Datenverbindung (V) zwischen dem Server (3) und dem Cloud-Dienst (5) vom Server (3) initiiert wird und der Cloud-Dienst (5) sich mittels eines Zertifikats authentisiert, wobei sich der Server (3) bevorzugt sich mit einem individuellen Berechtigungsnachweis authentisiert.

9. Verfahren nach einem der vorangehenden Ansprüche, wobei die Übermittlung des verschlüsselten Datensatzes (Dv) an den Cloud-Dienst (5) und/oder das Abrufen des verschlüsselten Datensatzes (Dv) von dem Cloud-Dienst (5) über eine https-Verbindung erfolgt.

10. Verfahren nach einem der vorangehenden Ansprüche, wobei der Cloud-Dienst (5) dem Benutzergerät (2) eine Publish- Subscribe-Schnittstelle zur Verfügung stellt und das Benutzergerät (2) bevorzugt dem Cloud-Dienst (5) bezüglich des gewünschten verschlüsselten Datensatzes (Dv) mit der Benutzer-Referenznummer (R) subskribiert.

11. Verfahren nach einem der vorangehenden Ansprüche, wobei das Benutzergerät (2) informiert wird, sobald der Server (3) den entsprechenden Datensatz (D) an den Cloud-Dienst (5) übermittelt hat.

12. System (1) zur sicheren Übertragung eines Datensatzes (D) zwischen einem Server (3) und einem Benutzergerät (2) mittels eines Cloud-Dienstes (5) umfassend einen Server (3), ein Benutzergerät (2) und eine Datenverbindung (V) zwischen Server (3) und Benutzergerät (2) und eine Datenschnittstelle (4) für eine Datenverbindung (V) zwischen Server (3) und Benutzergerät (2) mit einem Cloud-Dienst (5), wobei
- das Benutzergerät (2) zum Abrufen und/oder zur Erzeugung eines benutzerseitigen Diffie Hellman Schlüsselpaares (Bp, Bs) umfassend einen geheimen Benutzerschlüssel (Bs) und einen öffentlichen Benutzerschlüssel (Bp) ausgelegt ist,
- das System (1) zur Übermittlung des öffentlichen Benutzerschlüssels (Bp) an den Server (3) ausgelegt ist,
- der Server (3) zum Abrufen und/oder zur Erzeugung eines serverseitigen Diffie Hellman Schlüsselpaares (Sp, Ss) umfassend einen geheimen Serverschlüssel (Ss) und einen öffentlichen Serverschlüssel (Sp) ausgelegt ist,
- der Server (3) zum Abrufen eines Datensatzes (D) ausgelegt ist,
- der Server (3) zu einer Generierung eines serverseitigen Diffie Hellman Schlüssels (S1) unter Verwendung des geheimen Serverschlüssels (Ss) und des öffentlichen Benutzerschlüssels (Bp) und zur Verschlüsselung des Datensatzes (D) mittels des resultierenden serverseitigen Diffie Hellman Schlüssels (S1) ausgelegt ist,
- der Server (3) zur Übermittlung des verschlüsselten Datensatzes (Dv) an den Cloud-Dienst (5) ausgelegt ist,
- das Benutzergerät (2) zum Abrufen des öffentlichen Serverschlüssels (Sp) und zum Abrufen des verschlüsselten Datensatzes (Dv) von dem Cloud-Dienst (5) ausgelegt ist,
- das Benutzergerät (2) zur Generierung eines benutzerseitigen Diffie Hellman Schlüssels (S2) unter Verwendung des geheimen Benutzerschlüssels (Bs) und des abgerufenen öffentlichen Serverschlüssels (Sp) und zur Entschlüsselung des verschlüsselten Datensatzes (Dv) auf dem Benutzergerät (2) mit dem resultierenden benutzerseitigen Diffie Hellman Schlüssel (S2) ausgelegt ist.

13. Medizintechnische Anlage (6), bevorzugt zur Aufnahme und/oder zur Bearbeitung medizintechnischer Bilder, welches zur Integration als Server (3) in ein Verfahren nach einem der Ansprüche 1 bis 11 ausgelegt ist und/oder einen Server (3) von einem System (1) nach Anspruch 12 umfasst.

14. Computerprogrammprodukt mit einem Computerprogramm, welches direkt in eine Speichereinrichtung einer medizintechnischen Anlage (6) ladbar ist, mit Programmabschnitten, um alle Schritte des Verfahrens nach einem der Ansprüche 1 bis 11 auszuführen, wenn das Computerprogramm in der medizintechnischen Anlage (6) ausgeführt wird.

15. Computerlesbares Medium, auf welchem von einer Rechnereinheit einlesbare und ausführbare Programmabschnitte gespeichert sind, um alle Schritte des Verfahrens nach einem der Ansprüche 1 bis 11 auszuführen, wenn die Programmabschnitte von der Rechnereinheit ausgeführt werden.

## Claims

1. Method for the secure transfer of a dataset (D) between a server (3) and a user device (2) by means of a cloud service (5) comprising the steps:
- Provision or generation (I) of a user-side Diffie Hellman key pair (Bp, Bs) by the user device (2), comprising a secret user key (Bs) and a public user key (Bp),
- Transfer (II) of the public user key (Bp) to the server (3),
- Provision and/or generation (III) of a server-side Diffie Hellman key pair (Sp, Ss) by the server (3), comprising a secret server key (Ss) and a public server key (Sp),
- Provision (IV) of a dataset (D) on the server (3),
- Generation (V) of a server-side Diffie Hellman key (S1) on the server (3) by using the secret server key (Ss) and the public user key (Bp) and encryption of the dataset (D) on the server (3) by means of the resulting Diffie Hellman key (S1) on the server side,
- Transfer (VI) of the encrypted dataset (Dv) to the cloud service (5),
- Retrieval (VII) of the public server key (Sp) and retrieval of the encrypted dataset (Dv) from the cloud service (5) by means of the user device (2),
- Generation (VIII) of a user-side Diffie Hellman key (S2) on the user device by using the secret user key (Bs) and the retrieved public server key (Sp) and decryption of the encrypted dataset (Dv) on the user device (2) with the resulting Diffie Hellman key (S2) on the user side.

2. Method according to claim 1, wherein the encrypted dataset (Dv) together with the public server key (Sp) is transferred to the cloud service (5) and
the encrypted dataset (Dv) and the public server key (Sp) are also preferably stored together in the cloud service (5) or at least with a reference to one another,
wherein the encrypted dataset (Dv) together with the public server key (Sp) is preferably digitally signed by the server (3) .

3. Method according to one of the preceding claims, wherein in addition to the transfer of the public user key (Bp) a user reference number (R) is also transferred to the server (3) by the user device (2),
wherein the user reference number (R) is preferably also transferred as part of the transfer of the encrypted dataset (Dv) to the cloud service (5) and
the user reference number (R) and the encrypted dataset (Dv) are preferably stored together by the cloud service (5) or at least with a reference to one another.

4. Method according to one of the preceding claims, wherein a previously generated server-side Diffie Hellman key pair (Ss, Sp) for a defined number of datasets (D) and/or for a group of user devices (2) is used by the server (3).

5. Method according to one of the preceding claims, wherein in the context of the retrieval of the encrypted dataset (Dv) said dataset is retrieved together with the public server key (Sp) by the user device (2) from the cloud service (5), preferably indicating the user reference number (R).

6. Method according to one of the preceding claims, wherein once the public user key (Bp) has been transferred to the server (3) said server carries out an authentication of the user device (2), preferably by means of a challenge-response method, and in the event of a successful authentication continues with the provision or generation of a server-side Diffie Hellman key pair (Ss, Sp) by the server (3),
wherein an authorization of the user device (2) is preferably also checked.

7. Method according to one of the preceding claims, wherein a key derivation is performed by means of a key derivation function, preferably in accordance with a method of the group comprising PBKDF, HKDF, HMAC-SHA256, before encryption of the dataset (D) by the server (3),
wherein information and/or characteristics of the dataset designation preferably feed into the key derivation function.

8. Method according to one of the preceding claims, wherein for the transfer of the encrypted dataset (Dv) and preferably also of the public server key (Sp) and/or of the user reference number (R) the data connection (V) between the server (3) and the cloud service (5) is initiated by the server (3) and the cloud service (5) authenticates itself by means of a certificate, wherein the server (3) preferably authenticates itself with an individual proof of entitlement.

9. Method according to one of the preceding claims, wherein the transfer of the encrypted dataset (Dv) to the cloud service (5) and/or the retrieval of the encrypted dataset (Dv) from the cloud service (5) takes place via an https connection.

10. Method according to one of the preceding claims, wherein the cloud service (5) makes a publish/subscribe interface available to the user device (2) and the user device (2) preferably subscribes to the cloud service (5) with regards to the desired encrypted dataset (Dv) with the user reference number (R).

11. Method according to one of the preceding claims, wherein the user device (2) is informed as soon as the server (3) has transferred the corresponding dataset (D) to the cloud service (5) .

12. System (1) for the secure transfer of a dataset (D) between a server (3) and a user device (2) by means of a cloud service (5) comprising a server (3), a user device (2) and a data connection (V) between the server (3) and the user device (2) and a data interface (4) for a data connection (V) between the server (3) and the user device (2) with a cloud service (5), wherein
- the user device (2) is designed to retrieve and/or generate a user-side Diffie Hellman key pair (Bp, Bs) comprising a secret user key (Bs) and a public user key (Bp),
- the system (1) is designed to transfer the public user key (Bp) to the server (3),
- the server (3) is designed to retrieve and/or generate a server-side Diffie Hellman key pair (Sp, Ss) comprising a secret server key (Ss) and a public server key (Sp),
- the server (3) is designed to retrieve a dataset (D),
- the server (3) is designed to generate a server-side Diffie Hellman key (S1) by using the secret server key (Ss) and the public user key (Bp) and to encrypt the dataset (D) by means of the resulting server-side Diffie Hellman key (S1),
- the server (3) is designed to transfer the encrypted dataset (Dv) to the cloud service (5),
- the user device (2) is designed to retrieve the public server key (Sp) and to retrieve the encrypted dataset (Dv) from the cloud service (5),
- the user device (2) is designed to generate a user-side Diffie Hellman key (S2) by using the secret user key (Bs) and the retrieved public server key (Sp) and to decrypt the encrypted dataset (Dv) on the user device (2) with the resulting user-side Diffie Hellman key (S2).

13. Medical system (6), preferably to capture and/or to process medical images, which is designed for integration as a server (3) in a method according to one of claims 1 to 11 and/or comprises a server (3) of a system (1) according to claim 12.

14. Computer program product having a computer program which can be directly loaded into a memory storage facility of a medical system (6), having program sections in order to carry out all the steps of the method according to one of claims 1 to 11 when the computer program is executed in the medical system (6).

15. Computer-readable medium on which program sections that can be read in and executed by a computer unit are stored, in order to carry out all the steps of the method according to one of claims 1 to 11 when the program sections are executed by the computer unit.

## Revendications

1. Procédé de transmission sécurisée d'un ensemble (D) de données entre un serveur (3) et un appareil (2) utilisateur au moyen d'un service (5) de nuage, comprenant les stades :
- mise à disposition ou production (I) d'une paire (Bp, Bs) de clés de Diffie Hellman du côté de l'utilisateur par l'appareil (2) d'utilisateur, comprenant une clé (Bs) d'utilisateur privée et une clé (Bp) d'utilisateur publique,
- transmission (II) de la clé (Bp) d'utilisateur publique au serveur (3),
- mise à disposition et/ou production (III) d'une paire (Sp, Ss) de clés de Diffie Hellman du côté du serveur par le serveur (3) comprenant une clé (Ss) de serveur privée et une clé (Sp) de serveur publique,
- mise à disposition (IV) d'un ensemble (D) de données sur le serveur (3),
- création (V) d'une clé (S1) de Diffie Hellman du côté du serveur sur le serveur (3), en utilisant la clé (Ss) de serveur privée et la clé (Bp) d'utilisateur publique et chiffrement de l'ensemble (D) de données sur le serveur (3) au moyen de la clé (S1) de Diffie Hellman résultante du côté du serveur,
- transmission (VI) de l'ensemble (Dv) de données chiffrées au service (5) de nuage,
- appel (VII) de la clé (Sp) de serveur publique et appel de l'ensemble (Dv) de données chiffré par le service (5) de nuage au moyen de l'appareil (2) d'utilisateur,
- création (VIII) d'une clé (S2) de Diffie Hellman du côté de l'utilisateur sur l'appareil d'utilisateur, en utilisant la clé (Bs) d'utilisateur privée et la clé (Sp) de serveur publique appelée et déchiffrement de l'ensemble (Dv) de données chiffré sur l'appareil (2) d'utilisateur par la clé (S2) de Diffie Hellman résultante du côté de l'utilisateur.

2. Procédé suivant la revendication 1, dans lequel on transmet l'ensemble (Dv) de données chiffré, ensemble avec la clé (Sp) de serveur publique, au service (5) de nuage et,
de préférence, on met en mémoire l'ensemble (Dv) de données chiffré et la clé (Sp) de serveur publique dans le service (5) de nuage, ensemble ou au moins avec une référence l'un à l'autre,
dans lequel, de préférence, l'ensemble (Dv) de données chiffré, ensemble avec la clé (Sp) de serveur publique, est signée numériquement par le serveur (3).

3. Procédé suivant l'une des revendications précédentes, dans lequel supplémentairement à la transmission de la clé (Bp) d'utilisateur publique, il est produit également une transmission d'un numéro (R) de référence d'utilisateur par l'appareil (2) d'utilisateur au serveur (3),
dans lequel, de préférence, dans le cadre de la transmission de l'ensemble (Dv) de données chiffré au service (5) de nuage, on transmet supplémentairement le numéro (R) de référence d'utilisateur et,
de préférence, le numéro (R) de référence d'utilisateur et l'ensemble (Dv) de données chiffré sont mis en mémoire par le service (5) de nuage ensemble ou au moins avec une référence l'un à l'autre.

4. Procédé suivant l'une des revendications précédentes, dans lequel il est utilisé par le serveur (3) une paire (Ss, Sp) de clés de Diffie Hellman du côté du serveur élaborée précédemment pour un nombre défini d'ensemble (D) de données et/ou pour un groupe d'appareils (2) d'utilisateur.

5. Procédé suivant l'une des revendications précédentes, dans lequel dans le cadre de l'appel de l'ensemble (Dv) de données chiffré celui-ci, ensemble avec la clé (Sp) de serveur publique, est appelé du service (5) de nuage par l'appareil (2) d'utilisateur, de préférence en indiquant le numéro (R) de référence d'utilisateur.

6. Procédé suivant l'une des revendications précédentes, dans lequel, après la transmission de la clé (Bp) d'utilisateur publique au serveur (3), celui-ci effectue une authentification de l'appareil (2) d'utilisateur, de préférence par un procédé Challenge Response et, si l'authentification est couronnée de succès, on poursuit par le serveur (3) à la mise à disposition de la production d'une paire (Ss, Sp) de clés de Diffie Hellman du côté du serveur, dans lequel, de préférence, on contrôle supplémentairement aussi une autorisation de l'appareil (2) d'utilisateur.

7. Procédé suivant l'une des revendications précédentes, dans lequel, avant le chiffrement de l'ensemble (D) de données par le serveur (3), on effectue une déduction de clé par une fonction de déduction de clé, de préférence par un procédé du groupe PBKDF, HKDF, HMAC-SHA256,
dans lequel, de préférence, entre dans la fonction de déduction de clé des informations et/ou des caractéristiques de la désignation de l'ensemble de données.

8. Procédé suivant l'une des revendications précédentes, dans lequel, pour la transmission de l'ensemble (Dv) de données chiffré, et de préférence également aussi de la clé (Sp) de serveur public et/ou du numéro (R) de référence d'utilisateur, la liaison (V) de données entre le serveur (3) et le service (5) de nuage est lancée par le serveur (3) et le service (5) de nuage s'identifie au moyen d'un certificat, le serveur (3) s'identifiant de préférence par une preuve individuelle d'autorisation.

9. Procédé suivant l'une des revendications précédentes, dans lequel la transmission de l'ensemble (Dv) de données chiffré au service (5) de nuage et/ou l'appel de l'ensemble (Dv) de données chiffré par le service (5) de nuage a lieu par une liaison https.

10. Procédé suivant l'une des revendications précédentes, dans lequel le service (5) de nuage met à disposition de l'appareil (2) d'utilisateur une interface Publish-Subscribe et l'appareil (2) d'utilisateur souscrit, de préférence auprès du service (5) de nuage, en ce qui concerne l'ensemble (Dv) de données chiffré souhaité, par le numéro (R) de référence d'utilisateur.

11. Procédé suivant l'une des revendications précédentes, dans lequel l'appareil (2) d'utilisateur est informé dès que le serveur (3) a transmis l'ensemble (D) de données correspondant au service (5) de nuage.

12. Système (1) de transmission sécurisée d'un ensemble (D) de données entre un serveur (3) et un appareil (2) d'utilisateur au moyen d'un service (5) de nuage, comprenant un serveur (3), un appareil (2) d'utilisateur et une liaison (V) de données entre le serveur (3) et l'appareil (2) d'utilisateur et une interface (4) de données pour une liaison (V) de données entre le serveur (3) et l'appareil (2) d'utilisateur par un service (5) de nuage, dans lequel
- l'appareil (2) d'utilisateur est conçu pour l'appel et/ou la production d'une paire (Bp, Bs) de clés de Diffie Hellman du côté de l'utilisateur, comprenant une clé (Bs) d'utilisateur privée et une clé (Bp) d'utilisateur public,
- le système (1) est conçu pour la transmission de la clé (Bp) d'utilisateur publique au serveur (3),
- le serveur (3) est conçu pour l'appel et/ou la production d'une paire (Sp, Ss) de clés de Diffie Hellman du côté du serveur, comprenant une clé (Ss) de serveur privé et une clé (Sp) de serveur publique,
- le serveur (3) est conçu pour l'appel d'un ensemble (D) de données,
- le serveur (3) est conçu pour une création d'une clé (S1) de Diffie Hellman du côté du serveur, en utilisant la clé (Ss) de serveur privé et la clé (Bp) d'utilisateur publique, et pour le chiffrement de l'ensemble (D) de données au moyen de la clé (S1) de Diffie Hellman résultante du côté du serveur,
- le serveur (3) est conçu pour la transmission de l'ensemble (Dv) de données chiffré au service (5) de nuage,
- l'appareil (2) d'utilisateur est conçu pour l'appel de la clé (Sp) de serveur publique et pour l'appel de l'ensemble (Dv) de données chiffré par le service (5) de nuage,
- l'appareil (2) d'utilisateur est conçu pour la création d'une clé (S2) de Diffie Hellman du côté de l'utilisateur en utilisant la clé (Bs) d'utilisateur privée et la clé (Sp) de serveur publique appelée et pour le déchiffrement de l'ensemble (Dv) de données chiffré sur l'appareil (2) d'utilisateur par la clé (S2) de Diffie Hellman résultante du côté de l'utilisateur.

13. Installation (6) de la technique médicale, de préférence pour l'enregistrement et/ou le traitement d'images de la technique médicale, qui est conçue pour être intégrée comme serveur (3) à un procédé suivant l'une des revendications 1 à 11, et/ou à un serveur (3) d'un système (1) suivant la revendication 12.

14. Produit de programme d'ordinateur, ayant un programme d'ordinateur qui peut être chargé directement dans un dispositif de mise en mémoire d'une installation (6) de la technique médicale, comprenant des parties de programme pour effectuer tous les stades du procédé suivant l'une des revendications 1 à 11, lorsque le programme d'ordinateur est réalisé dans l'installation (6) de la technique médicale.

15. Support déchiffrable par ordinateur, sur lequel sont mises en mémoire des parties de programme pouvant être déchiffrées et réalisées par une unité d'ordinateur, afin d'effectuer tous les stades du procédé suivant l'une des revendications 1 à 11, lorsque les parties de programme sont réalisées par l'unité d'ordinateur.
